# EUROPEAN PATENT APPLICATION

(11) **EP 3 733 433 A1**
(43) Date of publication of application: **04.11.2020**
(21) Application number: 20167712.7
(22) Date of filing: 02.04.2020
(51) Int. Cl.: B60H 1/00, F24F 9/00, G06Q 50/30, G08G 1/0967, E04H 1/12, F24F 3/16

(54) **SYSTEM OF MONITORING AND IMPROVING AIR QUALITY ACCORDING TO A BUS SERVICE ROUTE AND CONTROL METHOD THEREOF**

(30) Priority: 29.04.2019 KR 20190049903
(71) Applicant: Korea Institute Of Civil Engineering And Building Technology, Goyang-si, Gyeonggi-do 10223 (KR)
(72) Inventor: LEE, SangHyup, Seoul (KR)
(74) Representative: Pons

(57) **Abstract**

A system (10) of monitoring and improving air quality according to a bus service route comprises a bus (100) which comprises a first apparatus (101) for measuring and improving air quality, a bus station (200) which comprises a second apparatus (201) for measuring and improving air quality, and a control system (300) which monitors and controls air quality data transferred from the first apparatus (101) and the second apparatus (201), and the control system (300) collects air quality material of the bus and the bus station in real time, and provides air quality information to the bus (100) and the bus station (200).

## Description

### TECHNICAL FIELD

The present invention relates to a system of monitoring and improving air quality and control method thereof, more specifically, to a system of monitoring and improving air quality according to a bus service route and control method thereof that can filter fine dust, etc. and monitor air quality along a bus service route.

### BACKGROUND ART

If looking at the present smart city technical trend of a transportation sector, Cooperative-Intelligent Transport Systems(C-ITS) technologies, etc. which utilize data collected through a transportation infra are developing drastically, but, from the view of technology application considering environment data, such as fine dust, etc., it is remarkably deficient.

Although the worries of related government departments and local governments continue in order to raise rate of public transportation use of citizens and lower rate of use of own car to solve the fine dust problems that increase rapidly every year, there are not the distinct solutions or immediate and effective response technologies and solutions, so vague anxiety of the policy decision managers, for sure, and of the citizens is getting bigger.

It is important to make citizens feel that they are in clean environment without fine dust through the visualization, etc. when they use the public transportations in order to make citizens be able to feel relieved to the fine dust damage and use the public transportations, but there are difficulties in the current technology and product situation.

Therefore, it is the best alternative to prepare the fine dust damage relief smart city application solution that can monitor the fine dust in real time in public transportation use environment and that can reduce it immediately in the case of "bad", and to install and operate in the proper position and time.

Accordingly, technical demand for air quality monitoring of the indoor and outdoor that can be applied to smart city is continually increasing from the local governments, the Ministry of Land, Infrastructure and Transport, related operators as supporting and managing agency of the fine dust response policy formulation including public transportation (bus) use activation policy, so it is urgent to put them into practical use at this time.

### Related Art Document

### Patent Document

Similar disclosures are shown in the prior art, for example, in Korean Patent No. KR 10-1771366, published August 24, 2017.

### DISCLOSURE

### Technical Problem

The present invention is to solve such problems, and an objective of the present invention is to provide a system of monitoring and improving air quality according to a bus service route and control method thereof that can monitor the fine dust data of a bus station and a bus on a regular route in real time and reduce immediately.

On the other hand, the objective of the present invention is to provide a system of monitoring and improving air quality according to a bus service route and control method thereof that make people feel relieved from fine dust damage increasing rapidly every year and take public transportation by establishing a system that can integrate and control data collected from it.

### Technical Solution

In order to achieve the above objects, according to one embodiment of the present invention, a system of monitoring and improving air quality according to a bus service route comprises a bus which comprises a first apparatus to measure and improve air quality, a bus station which comprises a second apparatus to measure and improve air quality, and a control system which monitors and controls air quality data transferred from the first apparatus and the second apparatus, wherein the control system which collects air quality material on the bus and the bus station in real time and provides air quality information to the bus and the bus station.

Also, the first apparatus can comprise a first inner air quality measuring instrument which measures air quality of inside the bus, a first outer air quality measuring instrument which measures air quality of outside the bus, a first air cleaner which filters dust in the air inside the bus, a first air quality monitor which guides air quality in real time measured by the first inner air quality measuring instrument and the first outer air quality measuring instrument, a first observation camera which observes a situation of inside the bus, and a first communicator to transmit first data about air quality measured by the first inner air quality measuring instrument and the first outer air quality measuring instrument to the control system.

Also, the second apparatus can comprise an air curtain which diverges air to prevent air of outside the bus station from flowing into inside the bus station, a second inner air quality measuring instrument which measures air quality of inside the bus station, a second outer air quality measuring instrument which measures air quality of outside the bus station, a second air cleaner which filters dust in the air inside the bus station, a second air quality monitor which guides air quality in real time measured by the second inner air quality measuring instrument and the second outer air quality measuring instrument, a second observation camera which observes a situation of inside the bus station, and a second communicator to transmit second data about air quality measured by the second inner air quality measuring instrument and the second outer air quality measuring instrument to the control system.

Also, the air curtain can comprise a suction part which inhales outer air, a filtration part which filters pollutants in the air inhaled by the suction part, a duct where air which passes the filtration part flows, a blower fan which transfers air which is accepted inside the duct and is filtered to the top of the duct, and a nozzle which sprays air transferred to the top of the duct through the blower fan.

Also, the control system can comprise a third communicator which communicates with the first communicator and the second communicator each other and receives the first data and the second data, a collector which collects air quality measurement information from the first data and the second data, an analyzer which analyzes air quality from the first data and the second data collected by the collector, a statistics processor which writes statistics about air quality analyzed by the analyzer, a visualization processor which visually represents the statistics written by the statistics processor, and a predictor which predicts data about air quality on a bus service route using analyzed data by the analyzer.

Also, the control system can control the first air cleaner and the second air cleaner to be operated respectively when air quality of inside the bus and inside the bus station is expressed lower than specific figure.

Also, the control system can further comprise a video analyzer which analyzes a video filmed by the first observation camera and the second observation camera.

Also, statistics about air quality analyzed by the control system and data about air quality predicted on a bus service route are provided to a user device.

In order to achieve the above objects, according to one embodiment of the present invention, a control method of a system of monitoring and improving air quality according to a bus service route can comprise a step to measure air quality of inside and outside a bus through a first apparatus prepared in the bus, a step to measure air quality of inside and outside a bus station through a second apparatus prepared in the bus station, a step to analyze air quality data transferred from the first apparatus and the second apparatus, a step to predict data about air quality on a bus service route using the analyzed air quality data, and a step to express the analyzed air quality data and the predicted air quality data on monitors prepared in the bus and the bus station.

Also, the control method can further comprise a step to control the first air cleaner installed inside the bus and the second air cleaner installed inside the bus station to be operated respectively when air quality of inside the bus and the bus station is expressed lower than specific figure.

Also, the control method can comprise a step to compare and analyze air quality of outside the bus and air quality of outside the bus station with traffic volume, and a step to express comparatively analyzed data on monitors prepared in the bus and the bus station.

### Advantageous Effects

According to the present invention, a system of monitoring and improving air quality according to a bus service route and control method thereof can improve air quality such as fine dust of the downtown by improving air quality of a bus service route including a bus and a bus station, by collecting, processing and providing materials about it to citizens, and by activating use of public transportation such as bus etc., and by restraining use of own car.

Also, as active and preemptive response to fine dust damage is possible, supply of a system for improving air quality can increase, so it is possible to implement a smart city.

### BRIEF DESCRIPTION OF DRAWINGS

FIG. 1 is a conceptual diagram of a system of monitoring and improving air quality according to a bus service route according to one embodiment of the present invention
FIG. 2 is a diagram of a device which is prepared in a bus shown in FIG. 1.
FIG. 3 is a diagram of a device which is prepared in a bus station shown in FIG. 1.
FIG. 4 is a diagram of an air curtain which is installed in the bus station shown in FIG. 3.
FIG. 5 is a diagram of a device which is prepared in the control system shown in FIG. 1.
FIG. 6 is a flow chart which expresses a control method of a system of monitoring and improving air quality according to a bus service route according to one embodiment of the present invention.

### *Reference numeral*

- 10:: a system of monitoring and improving air quality
- 100:: a bus
- 110:: a first inner air quality measuring instrument
- 120:: a first outer air quality measuring instrument
- 130:: a first air cleaner
- 140:: a first air quality monitor
- 150:: a first observation camera
- 160:: a first communicator
- 200:: a bus station
- 210:: an air curtain
- 220:: a second inner air quality measuring instrument
- 230:: a second outer air quality measuring instrument
- 240:: a second air cleaner
- 250:: a second air quality monitor
- 260:: a second observation camera
- 270:: a second communicator
- 300:: a control system

### Mode for Invention

Hereinafter, with reference to the accompanying drawings, a system of monitoring and improving air quality according to a bus service route and control method thereof according to embodiment of the present invention will be described with reference to the accompanying drawings. Thickness of lines shown in drawings and size of components, etc. may be shown exaggeratedly for clearness of explanation and convenience in the process. Also, the terms that follow, as terms defined considering the function of the present invention, may be different according to the intention or the convention of users and operators. Therefore, definition of the terms has to be defined on the base of the overall contents of the specification.

Also, a following embodiment does not limit scope of rights of the present invention, but just provides as an example, there can be various embodiments implemented through the present technical thought.

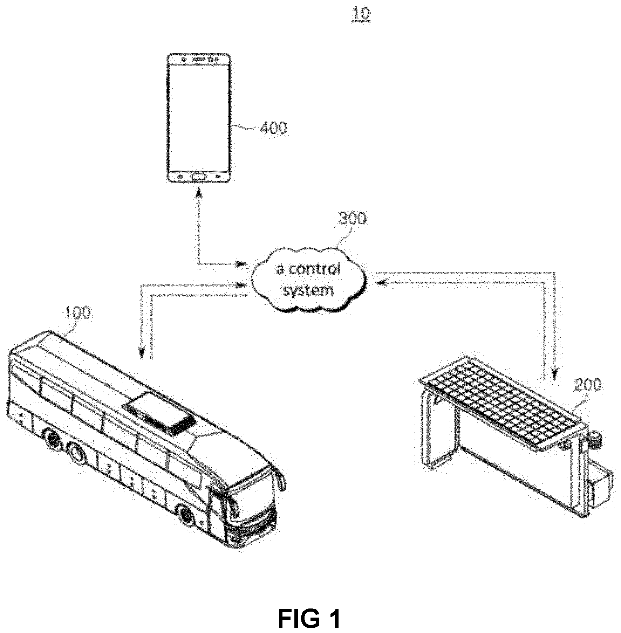

FIG. 1 is a conceptual diagram of a system of monitoring and improving air quality according to a bus service route according to one embodiment of the present invention.

A system of monitoring and improving air quality according to a bus service route (10) according to one embodiment of the present invention comprises a bus (100) which prepares a first apparatus (101) for measuring and improving air quality, a bus station (200) which prepares a second apparatus (201) for measuring and improving air quality, and a control system (300) which monitors and controls air quality data transferred from a first apparatus (101) and a second apparatus (201).

The control system (300) collects air quality materials of the bus (100) and the bus station (200) in real time and provides air quality information to the bus (100) and the bus station (200).

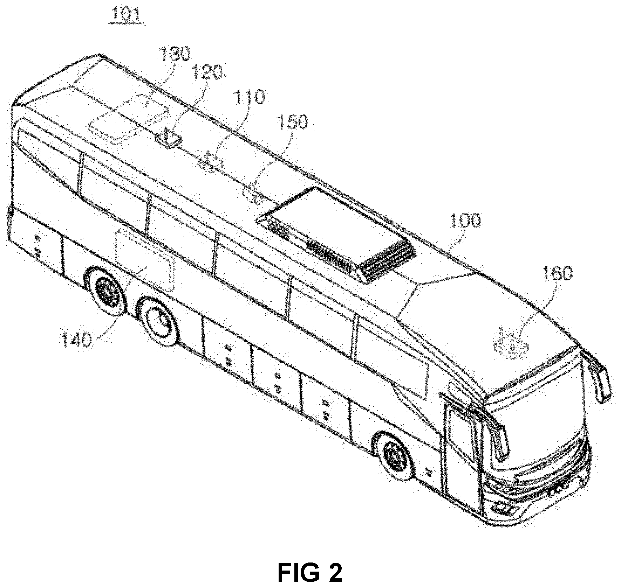

FIG. 2 is a diagram of a device which is prepared in the bus shown in FIG. 1.

With reference to the FIG. 2, a first apparatus (101) comprises a first inner air quality measuring instrument (110) which measures air quality of inside the bus (100), a first outer air quality measuring instrument (120) which measures air quality of outside the bus (100), a first air cleaner (130) which filters dust in the air inside the bus (100), a first air quality monitor (140) which guides air quality in real time measured by the first inner air quality measuring instrument (110) and the first outer air quality measuring instrument (120), a first observation camera (150) which observes a situation of inside the bus (100), and a first communicator (160) to transmit first data about air quality measured by the first inner air quality measuring instrument (110) and the first outer air quality measuring instrument (120) to the control system (300).

The bus (100) moves along decided bus route, in particularly a bus route is formed between buildings a lot in the downtown, and the bus moves along the corridor. Recently fine dust occurs a lot and smoke occurring in a diesel car is chosen as a cause of the fine dust. Therefore, concentration of the fine dust may be high on a bus route with many cars.

The bus (100) comprises the first inner air quality measuring instrument (110) which measures air quality of inside the bus (100), and the first outer air quality measuring instrument (120) which measures air quality of outside the bus (100) and can measure air quality of inside and outside the bus (100). The first inner air quality measuring instrument (110) and the first outer air quality measuring instrument (120) measure air quality of inside and outside the bus (100) in real time, as the bus (100) moves. Therefore, air quality along a bus route is figured out in real time.

On the other hand, a first air cleaner (130) which filters dust, etc. in the air inside the bus (100) when air quality in the bus (100) is not good, is prepared in the bus (100). The first air cleaner (130) can eliminate temperature, humidity, concentration of carbon dioxide, dust, fine dust, VOCs, etc. which can be flown into inside the bus (100) when the bus (100) passes a bus route.

Also, a first air quality monitor (140) which guides air quality in real time measured by the first inner air quality measuring instrument (110) and the first outer air quality measuring instrument (120) is prepared inside the bus (100). Therefore, bus (100) passengers can see the first air quality monitor (140) and can check outdoor air quality on the bus route where the bus (100) which they are taking in current time goes, and indoor air quality in the bus in real time, and thus it can enable the passengers to take an action against the indoor and outdoor air quality.

Meanwhile, a first observation camera (150) which monitors a situation of inside the bus (100) is prepared. The first observation camera (150) can observe whether fine dust, etc. are flow inside the bus (100) by monitoring a situation, etc. of inside the bus (100).

Also, a first communicator (160) to transmit first data about air quality measured by a first inner air quality measuring instrument (110) and a first outer air quality measuring instrument (120) to the control system (300) is prepared. Therefore, first data about air quality measured by the first inner air quality measuring instrument (110) and the first outer air quality measuring instrument (120) is transmitted to the control system (300) and from now on the control system (300) can provide air quality data to the first air quality monitor (140) using the first communicator (160) and provide signal to operate the first air cleaner (130).

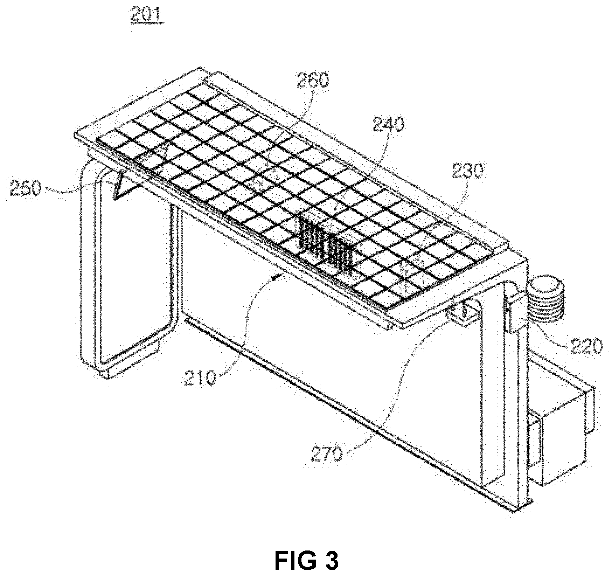

FIG. 3 is a diagram of a device which is prepared in the bus station shown in FIG. 1.

With the reference to the FIG. 3, the second apparatus (201) comprises an air curtain (210) which diverges air to prevent outer air of the bus station (200) from flowing into inside the bus station (200), a second inner air quality measuring instrument (220) which measures air quality of inside the bus station (200), a second outer air quality measuring instrument (230) which measures air quality of the outside the bus station (200), a second air cleaner (240) which filters dust in the air inside the bus station (200), a second air quality monitor (250) which guides air quality in real time measured by the second inner air quality measuring instrument (220) and the second outer air quality measuring instrument (230), a second observation camera (260) which observes a situation of inside the bus station (200), and a second communicator (270) to transmit second data about air quality measured by the second inner air quality measuring instrument (220) and the second outer air quality measuring instrument (230) to the control system (300).

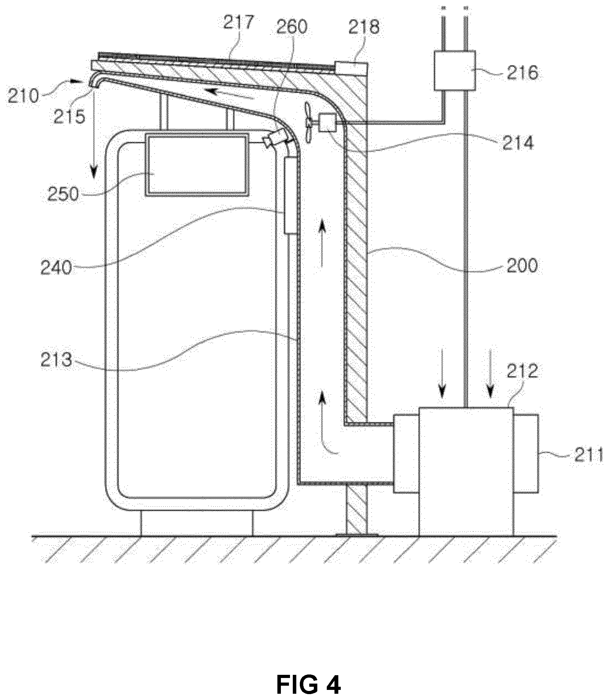

FIG. 4 is a diagram of an air curtain which is installed in the bus station shown in FIG. 3.

With the reference to the FIG. 4, as a device which blows wind to distinguish inside and outside the bus station (200), the air curtain (210) plays a role which prevents fine dust of roadside from flowing into inside the bus station (200) by distinguishing inside and outside the bus station (200).

The air curtain (210) prepared in bus station (200) according to the present embodiment comprises a suction part (211) which inhales outer air, a filtration part (212) which filters pollutants in the air inhaled by the suction part (210), a duct (213) where the air which passes filtration part (212) flows, a blower fan (214) which transfers the air which is accepted inside the duct (213) and is filtered to the top of the duct (213), and a nozzle (215) which sprays air transferred to the top of the duct through the blower fan (214).

The suction part (211) is a place to introduce outer air of the bus station (200) into an inside thereof. Meanwhile, the air introduced through suction part (211) is introduced to inner side of the bus station through the filtration part (212), and as a filter is installed in the filtration part (212), foreign substances such as dust, etc. in the air passing the filtration part are removed.

After the air passing the filtration part (212) is guided to the duct (213) of part which forms the roof of the bus station (200), it is sprayed through the nozzle (215) formed in the end of duct (213). At this time, as the nozzle (215) has narrow flowing road, the air sprayed through the nozzle (215) has high pressure and can reach bottom of the bus station (200). If the air sprayed through the nozzle (215) reaches the bottom of the bus station (200), polluted air on the road cannot be flown into inside the bus station (200) because the air curtain is formed.

On the other hand, the blower fan (214) of the air curtain (210) can be controlled by a controlling part (216). For example, the controlling part (216) doesn't operate the air curtain (210) when air quality of outside the bus station (200) is good, and can operate the air curtain (210) when air quality of outside the bus station (200) is not good.

On the other hand, a solar power unit (217) and storage device (218) which stores generated electricity are prepared on the ceiling of the bus station (200) and can supply electricity spent in the blower fan (214).

Inside and outside the bus station (200) are segregated, and air quality of the inside the bus station (200) can be administered by preparing the air curtain (210).

With the reference to the FIG. 3 again, the bus station (200) comprises the second air quality measuring instrument (220) which measures air quality of inside the bus station (200), and the second outer air quality measuring instrument (230) which measures air quality of outside the bus station (200) and air quality of inside and outside the bus station (200) can be measured. The second inner air quality measuring instrument (220) and the second outer air quality measuring instrument (230) measure air quality of inside the bus station (200) in real time. Therefore, air quality according to bus location, etc. can be figured out in real time when the bus (100) moves along a bus route.

On the other hand, the second air cleaner (240) which filters dust, etc. in the air of inside the bus station (200) when air quality of inside the bus station (200) is not good, is prepared in the bus station (200). The second air cleaner (240) can eliminate temperature, humidity, concentration of carbon dioxide, dust, fine dust, VOCs, and etc. which can flow into inside the bus station (200), when the bus (100) passes a bus route.

Also, the second air quality monitor (250) which guides air quality in real time measured by the second inner air quality measuring instrument (220) and the second outer air quality measuring instrument (230) is prepared in the bus station (200). Therefore, people who are waiting in the bus station (200) see the second air quality monitor (250) and can check the outdoor air quality of the bus station (200) where they are waiting in current time and indoor air quality of the bus station (200) in real time.

Meanwhile, the second observation camera (260) which monitors a situation of inside the bus station (200) is prepared. The second observation camera (260) can observe whether fine dust, etc. flow into the bus station (200) by monitoring a situation, etc. of inside the bus station (200).

Also, the second communicator (270) is prepared to transmit second data about air quality measured by the second inner air quality measuring instrument (220) and the second outer air quality measuring instrument (230) to the control system (300). Therefore, the second data about air quality measured by the second inner air quality measuring instrument (220) and the second outer air quality measuring instrument (230) is transmitted to the control system (300), and after that the control system (300) can provide air quality data to the second air quality monitor (250) using the second communicator (270), and can provide a signal to operate the second air cleaner (240).

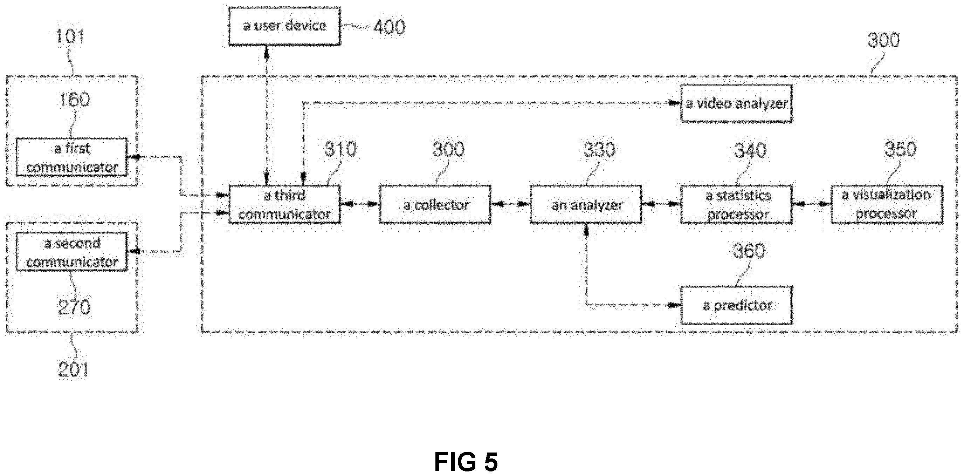

FIG. 5 is a diagram of a device which prepared in the control system shown in FIG. 1.

With reference to the FIG. 5 the control system (300) comprises a third communicator (310) which receives the first data and the second data by communicating with the first communicator (160) and the second communicator (270) each other, a collector (320) which collects air quality measurement information from the first data and the second data, an analyzer (330) which analyzes air quality from the first data and the second data collected by the collector (320), a statistics processor (340) which writes statistics about air quality analyzed by the analyzer (330), a visualization processor (350) which visually represents the statistics written by the statistics processor (340), and a predictor (360) which predicts data about air quality on a bus service route using analyzed data by the analyzer (330).

The third communicator (310) receives various data measured in a bus (100) and a bus station (200) through the first communicator (160) installed in the bus (100) and the second communicator (270) installed in the bus station (200).

The collector (320) collects air quality measurement information from the first data and the second data. At this time, the received air quality measurement information can be various information related to air quality such as temperature, humidity, concentration of carbon dioxide, dust, fine dust, VOCs, and etc.

The analyzer (330) analyzes air quality from the first data and the second data collected by collector (320).

The statistics processor (340) writes statistics about air quality analyzed by the analyzer (330). For example, the statistics can be written about which of temperature, humidity, concentration of carbon dioxide, dust, fine dust, VOCs is high and low in each section of a bus route.

The visualization processor (350) visually represents the statistics written by the statistics processor (340). So, after the statistics written by the statistics processor (340) is visually represented, which can be provided to the first air quality monitor (140) prepared in the bus (100) and the second air quality monitor (250) prepared in the bus station (200). Therefore, citizens can figure out easily air quality according to a bus service route by visually providing air quality along a bus route to the citizens who are taking the bus.

The predictor (360) predicts data about air quality on a bus service route using analyzed data by the analyzer (330). When the predictor (360) predicts air quality in a bus service route, it receives information about wind, temperature, humidity from Meteorological Agency, and information about traffic volume, etc. on a bus service route and can predict data about air quality of a bus service route. So, the predicted data about air quality can be provided to the first air quality monitor (140) prepared in the bus (100) and the second air quality monitor (250) prepared in the bus station (200). Therefore, by providing air quality predicted along a bus route to citizens who are using the bus, when the citizens use public transportation according to a bus service route, it enables citizens to prepare tools like mask beforehand.

Meanwhile, when air quality of inside the bus (100) and bus station (200) is expressed lower than specific figure, the control system (300) can control the first air cleaner (130) and the second air cleaner (240) respectively to be operated. For example, when air quality of inside the 10^{th} bus which runs according to a bus service route is expressed lower than specific figure, the first air cleaner (130) which is installed in the 10^{th} bus can be operated without operation of operator. Therefore, it can be prevented for passengers using the bus (100) to be exposed to bad air.

When air quality of the 10^{th} bus station on a bus service route is expressed lower than specific figure, the second air cleaner (240) installed in the 10^{th} bus station can be operated. Therefore, it is prevented for passengers using the bus station (200) to be exposed to bad air. Meanwhile, when air quality of the bus station (200) on a bus service route is expressed bad, citizens located in the bus station (200) are prevented to be exposed to bad air by working the air curtain (210) so as to isolate the inside of the bus station (200) from the outside of the bus station (200).

Like this, by controlling air quality of inside the bus (100) or the bus station (200), by providing information about improved air quality through a monitor and by planting awareness that citizens are not exposed to bad air using a bus, bus use of citizens can be activated. Therefore, use of own car is reduced, which can contribute improvement of fine dust concentration.

Meanwhile, the control system (300) further comprises a video analyzer (370) which analyzes a video filmed by the first observation camera (150) and the second observation camera (260). Discomfort, etc. which citizens are feeling about air quality in the bus (100) or the bus station (200) can be observed through the video analyzer (370) and it can take an action against the above.

Also, the statistics about air quality analyzed in the control system (300) and data about air quality predicted on a bus service route are provided to a user device (400). Therefore, a bus user can check information about air quality provided to his/her device (400) and can use the public transportation like bus, etc. and can retain mask, etc. and prepare properly by figuring out information about air quality on a bus route beforehand.

To the next, a control method of a system of monitoring and improving air quality according to a bus service route is explained.

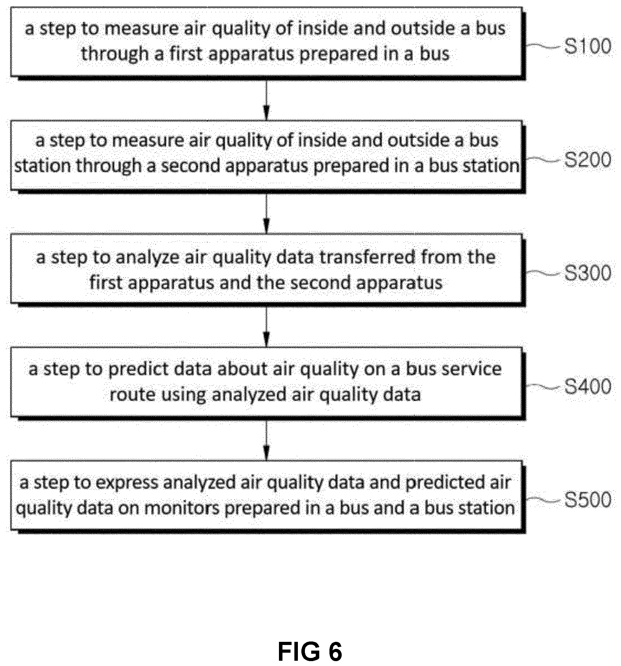

FIG.6 is a flow chart which expresses control method of a system of monitoring and improving air quality according to a bus service route according to one embodiment of the present invention.

With the reference to the FIG. 6, control method of a system of monitoring and improving air quality of a bus service route according to one embodiment of the present invention comprises a step (S100) to measure air quality of inside and outside the bus (100) through a first apparatus (101) prepared in the bus, a step (S200) to measure air quality of inside and outside the bus station (200) through a second apparatus (201) prepared in the bus station (200), a step (S300) to analyze air quality data transferred from the first apparatus (101) and the second apparatus (201), a step (S400) to predict data about air quality on a bus service route using the analyzed air quality data, and a step (S500) to express analyzed air quality data and the predicted air quality data on monitors (140, 250) prepared in the bus (100) and the bus station (200).

On the other hand, when air quality of inside the bus (100) and the bus station (200) is expressed lower than specific figure, the first air cleaner (130) installed in the bus (100) and the second air cleaner (240) installed in the bus station (200) are controlled to be operated respectively. Therefore, air quality of inside the bus (100) and the bus station (200) which citizens who take a bus mainly use can be secured well, good awareness about a bus can be planted to citizens, and accordingly rate of use of own car may decrease and use of a bus (100) may increase.

The control method of a system of monitoring and improving air quality of a bus service route further comprises a step to compare and analyze air quality of outside the bus (100) and the bus station (200) with traffic volume, and a step to express the comparatively analyzed data on monitors (140, 250) prepared in the bus (100) and the bus station (200).

Like this, if a step to compare and analyze air quality of outside the bus (100) and the bus station (200) with traffic volume and a step to express it are provided to citizens, a relation that air quality gets worse with an increasing traffic volume is provided to citizens, and then a rate of use of own car of citizens may decrease, such that it can contribute improvement of environment.

In the above, a system of monitoring and improving air quality according to a bus service route and control method thereof according to the present invention was described. By a system of monitoring and improving air quality according to a bus service route and control method thereof according to the present invention, use of public transportation like a bus, etc. is activated by improving air quality of a bus service route including a bus and a bus station and by collecting and processing materials about it and providing them to citizens, and use of own car is suppressed, such that the air quality such as fine dust of downtown, etc. may be improved.

Embodiments of the present invention described as above are only examples, those skilled in the art will be able to know readily that various modification, and equal another embodiment are possible. Thus, it is understood well that the present invention is not limited to the form which mentioned in the detailed description. Therefore, genuine technical scope of the present invention has to be determined by technical spirit of the invention of attached claims. Also, the present invention should be understood to comprise spirit of the present invention defined by attached claims and all variants, equivalents, and substitutions within the scope of the invention

## Claims

1. A system of monitoring and improving air quality according to a bus service route comprising:
a bus which comprises a first apparatus to measure and improve air quality,
a bus station which comprises a second apparatus to measure and improve air quality, and
a control system which monitors and controls air quality data transferred from the first apparatus and the second apparatus,
wherein the control system which collects air quality material of the bus and the bus station in real time and provides air quality information to the bus and the bus station.

2. The system of monitoring and improving air quality according to a bus service route according to Claim 1, the first apparatus comprises:
a first inner air quality measuring instrument which measures air quality of inside the bus,
a first outer air quality measuring instrument which measures air quality of outside the bus,
a first air cleaner which filters dust in the air inside the bus,
a first air quality monitor which guide air quality in real time measured by the first inner air quality measuring instrument and the first outer air quality measuring instrument,
a first observation camera which observes a situation of inside the bus, and
a first communicator to transmit first data about air quality measured by the first inner air quality measuring instrument and the first outer air quality measuring instrument to the control system.

3. The system of monitoring and improving air quality according to a bus service route according to Claim 2, the second apparatus comprises:
an air curtain which diverges air to prevent air of outside the bus station from flowing into inside the bus station,
a second inner air quality measuring instrument which measures air quality of inside the bus station,
a second outer air quality measuring instrument which measures air quality of outside the bus station,
a second air cleaner which filters dust in the air inside the bus station,
a second air quality monitor which guides air quality in real time measured by the second inner air quality measuring instrument and the second outer air quality measuring instrument,
a second observation camera which observes a situation of inside the bus station, and
a second communicator to transmit second data about air quality measured by the second inner air quality measuring instrument and the second outer air quality measuring instrument to the control system.

4. The system of monitoring and improving air quality according to a bus service route according to Claim 3, the air curtain comprises:
a suction part which inhales outer air,
a filtration part which filters pollutants in the air inhaled by the suction part,
a duct where air which passes the filtration part flows,
a blower fan which transfers air which is accepted inside the duct and filtered to the top of the duct, and
a nozzle which sprays air transferred to the top of the duct through the blower fan.

5. The system of monitoring and improving air quality according to a bus service route according to Claim 4, the control system comprises:
a third communicator which communicates with the first communicator and the second communicator each other and receives the first data and the second data,
a collector which collects air quality measurement information from the first data and the second data,
an analyzer which analyzes air quality from the first data and the second data collected by the collector,
a statistics processor which writes statistics about air quality analyzed by the analyzer,
a visualization processor which visually represents the statistics written by the statistics processor, and
a predictor which predicts data about air quality on a bus service route using analyzed data by the analyzer.

6. The system of monitoring and improving air quality according to a bus service route according to Claim 5, the control system controls the first air cleaner and the second air cleaner to be operated respectively when air quality of inside the bus and the bus station is expressed lower than specific figure.

7. The system of monitoring and improving air quality according to a bus service route according to Claim 5, the control system further comprises a video analyzer which analyzes a video filmed by the first observation camera and the second observation camera.

8. The system of monitoring and improving air quality according to a bus service route according to Claim 6, providing statistics about air quality analyzed in the control system and data about air quality predicted on a bus service route to a user device.

9. A control method of a system of monitoring and improving air quality according to a bus service route comprising,
a step to measure air quality of inside and outside a bus through a first apparatus prepared in the bus,
a step to measure air quality of inside and outside a bus station through a second apparatus prepared in the bus station,
a step to analyze air quality data transferred from the first apparatus and the second apparatus,
a step to predict data about air quality on a bus service route using the analyzed air quality data, and
a step to express the analyzed air quality data and the predicted air quality data on monitors prepared in the bus and the bus station.

10. The control method of a system of monitoring and improving air quality according to a bus service route according to Claim 9, further comprising a step to control the first air cleaner installed inside the bus and the second air cleaner installed inside the bus station to be operated respectively when air quality of inside the bus and the bus station is expressed lower than specific figure.

11. The control method of a system of monitoring and improving air quality according to a bus service route according to Claim 9, comprising a step to compare and analyze air quality of outside the bus and air quality of outside the bus station with traffic volume, and
a step to express the comparatively analyzed data on monitors prepared in the bus and the bus station.
